(19) Europäisches Patentamt
European Patent Office
Office européen des brevets

(11) Veröffentlichungsnummer: **0 008 438**
**A1**

# (12) EUROPÄISCHE PATENTANMELDUNG

(21) Anmeldenummer: 79102980.4

(22) Anmeldetag: 16.08.79

(51) Int. Cl.³: C 07 C 143/68
A 61 K 31/325

(30) Priorität: 19.08.78 DE 2836385

(43) Veröffentlichungstag der Anmeldung:
05.03.80 Patentblatt 80/5

(84) Benannte Vertragsstaaten:
AT BE CH DE FR GB IT NL SE

(71) Anmelder: HOECHST AKTIENGESELLSCHAFT
Zentrale Patentabteilung Postfach 80 03 20
D-6230 Frankfurt/Main 80(DE)

(72) Erfinder: Loewe, Heinz, Dr.
Berliner Ring 6
D-6233 Kelkheim (Taunus)(DE)

(72) Erfinder: Urbanietz, Josef
Am Sandring 44
D-6231 Schwalbach(DE)

(72) Erfinder: Düwel, Dieter, Dr.
Frankfurter Strasse 39
D-6238 Hofheim am Taunus(DE)

(72) Erfinder: Kirsch, Reinhard, Dr.
Kurfürstenstrasse 10
D-6239 Eppstein/Taunus(DE)

## (54) Monocarboxylate von Phenylguanidinsulfonsäureestern, Verfahren zu ihrer Herstellung und ihre Verwendung als Arzneimittel.

(57) Es werden neue Monocarboxylate von Phenylguanidinsulfonsäureestern beschrieben sowie Verfahren zu ihrer Herstellung. Sie wirken als Arzneimittel gegen Helminthen und Leberegeln sowohl oral als auch parenteral.

EP 0 008 438 A1

HOECHST AKTIENGESELLSCHAFT HOE 78/F 172 Dr.KM/jk

Monocarboxylate von Phenylguanidinsulfonsäureestern, Verfahren zu ihrer Herstellung und ihre Verwendung als Arzneimittel.

Es ist bekannt, daß Phenylguanidine der Formel

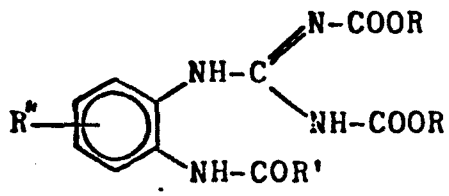

in der R niederes Alkyl, R' niederes Alkyl oder Wasserstoff und R" $-C_4H_9$, $-COC_6H_5$, $-OC_6H_5$ oder $-SC_6H_5$ bedeuten, anthelminthisch wirksam sind (vgl. DT-OS 21 17 293, 22 50 911, 23 04 764 und 24 23 679).

Diese Phenylguanidine und auch Anthelminthica aus der Klasse der Benzimidazol-2-carbaminate, wie Parbendazol, Mebendazol, Fenbendazol und andere Vertreter dieser Körperklasse sind in Wasser schwer löslich und können daher in der Human- und Veterinärmedizin nur oral appliziert werden. Mit diesen Stoffen lassen sich nicht ohne

weiteres parenteral anwendbare, örtlich gut verträgliche Lösungen herstellen, wie sie besonders für die Behandlung von Großtieren wie Rindern, Pferden und Schweinen benötigt werden.

Weiterin sind nach DT-OS 26 30 847 Methoxycarbonyl-phenylguanidine der Formel

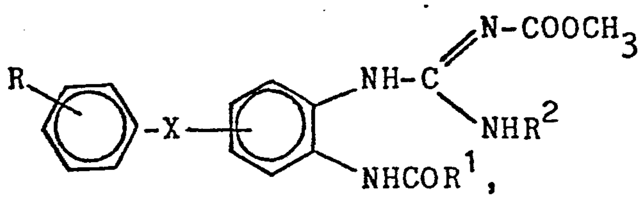

in der X für Sauerstoff, Schwefel, die -SO-, $-SO_2-$ oder -CO-Gruppe steht und in der X in Stellung 4 oder 5 mit dem Phenylguanidinorest verbunden ist, R Wasserstoff, Halogen, Alkoxy-($C_1$-$C_4$) oder Trifluormethyl, $R^1$ Wasserstoff, gegebenenfalls substituiertes Alkyl, gegebenenfalls substituiertes Alkoxy, gegebenenfalls substituiertes Alkoxyalkyl, gegebenenfalls substituiertes Aralkyl, $R^2$ Wasserstoff, Alkyl, Cycloalkyl, Cycloalkylalkyl oder Aralkyl bedeuten, bekannt, deren Salze mit anorganischen und organischen Säuren in Wasser löslich und damit parenteral anwendbar sind. Sie wirken zwar gegen ein breites Spektrum von Nematoden, jedoch nicht ausreichend gegen Leberegel, einen Parasiten, der häufig zusammen mit den Nematoden des Magen-Darm-Traktes vorkommt und zweckmäßig zusammen mit den Nematoden bekämpft wird.

Nach DT-OS 24 41 201 und 24 41 202 sind zwar Benzimidazol-carbaminate der Formel

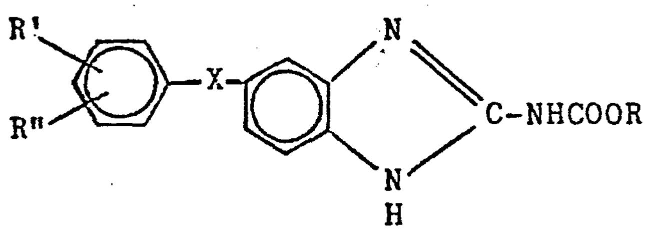

bekannt, in der X die Gruppe $-OSO_2-$ oder $-SO_2O-$, R Alkyl mit 1 bis 4 C-Atomen, R' und R" jeweils unabhängig voneinander Wasserstoff, Hydroxyl, Alkyl oder Alkoxy mit jeweils 1 - 4 C-Atomen, Halogen, Trifluoromethyl oder CN

bedeuten, und die sowohl gegen Nemathoden als auch gegen Leberegel wirksam, aber wegen der geringen Löslichkeit in Wasser nur oral applizierbar sind.

Gegenstand der Erfindung sind Monocarboxylate von Phenylguanidinsulfonsäureestern der Formel I

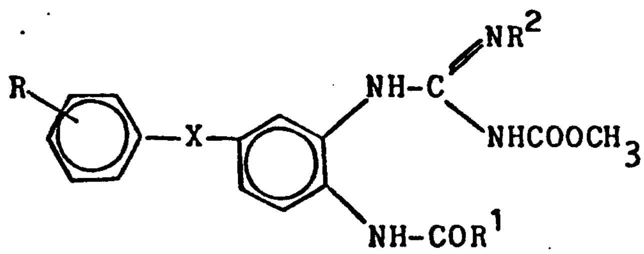

(I)

in welcher

X $-OSO_2-$ oder $-SO_2O-$,

R Wasserstoff, Halogen, Alkyl mit 1 - 4 C-Atomen, Alkoxy mit 1 - 4 C-Atomen, -CN oder $-CF_3$,

$R^1$ Wasserstoff, gegebenenfalls substituiertes Alkyl, gegebenenfalls substituiertes Alkoxy, gegebenenfalls substituiertes Alkoxyalkyl oder gegebenenfalls substituiertes Aralkyl und

$R^2$ Wasserstoff, Alkyl, Cycloalkyl, Cycloalkyl-alkyl oder Aralkyl bedeuten,

und ihre physiologisch verträglichen Salze mit organischen oder anorganischen Säuren.

Die erfindungsgemäßen Verbindungen der Formel I haben basischen Charakter. Sie können als freie Basen oder in Form ihrer Salze mit physiologisch verträglichen anorganischen und organischen Säuren, beispielsweise Hydrohalogenide, vorzugsweise Hydrochloride, Sulfate, Phosphate, Nitrate, Maleinate, Fumarate, Acetate, Propionate, Lactate, Methansulfonate oder Naphthalindisulfonate, als anthelminthische Wirkstoffe verwendet werden.

Gegenstand der Erfindung ist auch ein Verfahren zur Herstellung der Monocarboxylate von Phenylguanidinsulfonsäureestern der Formel I, das dadurch gekennzeichnet ist, daß man

a) ein substituiertes Anilinderivat der Formel II

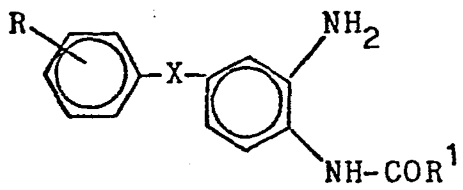

(II)

in welcher X, R und $R^1$ die zu Formel I angegebenen Bedeutungen haben, entweder

$a_1$) mit einem Isothioharnstoffderivat der Formel III

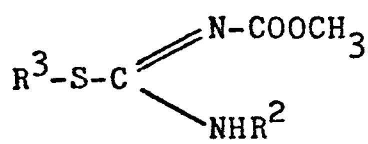

(III)

in welcher $R^2$ die zu Formel I angegebene Bedeutung hat und $R^3$ für Alkyl mit 1 - 4 C-Atomen steht,

oder

$a_2$) mit einem O-Methylisoharnstoffderivat der Formel IIIa

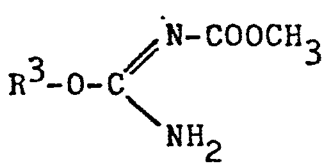

(IIIa)

in welcher $R^3$ für Alkyl mit 1 - 4 C-Atomen steht, in Gegenwart eines Lösungsmittels und gegebenenfalls in Gegenwart einer Säure umsetzt,

oder

b) ein Phenylguanidin-bis-carboxylat der Formel IV

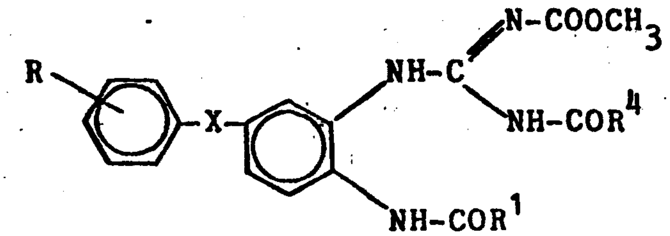

(IV)

in welcher
X, R und $R^1$ die oben angegebenen Bedeutungen haben und
$R^4$ für Alkyl oder Alkoxy steht, hydrolysiert,

und gegebenenfalls eine so erhaltene Verbindung der Formel I, in welcher $R^2$ Wasserstoff bedeutet, alkyliert.

In den Formeln I bis IV haben die Substituenten die folgenden bevorzugten Bedeutungen:
Als Halogen R steht Fluor, Chlor und Brom, insbesondere Fluor und Chlor.
Als Alkoxy-($C_1$-$C_4$) steht Methoxy, Äthoxy, n-Propoxy, i-Propoxy, n-Butoxy, i-Butoxy und t-Butoxy, vorzugsweise Methoxy und Äthoxy.
Als gegebenenfalls substituiertes Alkyl$R^1$ steht geradkettiges oder verzweigtes Alkyl mit vorzugsweise 1 bis 6, insbesondere 1 bis 4 Kohlenstoffatomen. Beispielshaft seien gegebenenfalls substituiertes Methyl, Äthyl, n- und i-Propyl, n- 1- und t-Butyl, genannt.
Als gegebenenfalls substituiertes Alkoxy $R^1$ steht geradkettiges oder verzweigtes Alkoxy mit vorzugsweise 1 bis 6, insbesondere 1 bis 4 Kohlenstoffatomen. Beispielshaft seien gegebenenfalls substituiertes Methoxy, Äthoxy, n- und i-Propoxy und n-, i- und t-Butoxy genannt.
Als gegebenenfalls substituiertges Alkoxyalkyl $R^1$ steht geradkettiges oder verzweigtes Alkoxyalkyl mit vorzugsweise 1 bis 6, insbesondere 1 bis 4, Kohlenstoffatomen im Alkoxyteil und vorzugsweise 1 bis 6, insbesondere 1 bis 4, Kohlenstoffatomen in Alkylteil. Beispielshaft seien gegebenenfalls substituiertes Methoxymethyl, Methoxyäthyl, Äthoxymethyl, Äthoxyäthyl genannt.

Als gegebenenfalls substituiertes Aralkyl $R^1$ steht gegebenenfalls im Arylteil und/oder Alkylteil substituiertes Aralkyl mit vorzugsweise 6 bis 10 Kohlenstoffatomen im Arylteil und vorzugsweise 1 bis 4, insbesondere 1 oder 2, Kohlenstoffatomen im Alkylteil, wobei der Alkylteil geradkettig oder verzweigt sein kann. Beispielshaft seien gebenenfalls substituiertes Benzyl und Phenyläthyl genannt.

Die verschiedenen Reste $R^1$ können einen oder mehrere, vorzugsweise 1 bis 3, insbesondere 1 oder 2 gleiche oder verschiedene Substituenten tragen. Als Substituenten seien beispielshaft aufgeführt:

Alkoxy mit vorzugsweise 1 bis 4, insbesondere 1 oder 2 Kohlenstoffatomen, wie Methoxy, Äthoxy, n- und i-Propyloxy und n-, i- und t-Butyloxy; Alkylthio mit vorzugsweise 1 bis 4, insbesondere 1 oder 2 Kohlenstoffatomen, wie Methylthio, Äthylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1. bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor und Chlor; Cyano; Nitro; Amino; Monoalkyl- und Dialkylamino mit vorzugsweise 1 bis 4, insbesondere 1 oder 2 Kohlenstoffatomen je Alkylgrupe, wie Methylamino, Methyl-äthyl-amino, n- und i-Propylamino und Methyl-n-Butylamino; Carbalkoxy mit vorzugsweise 2 bis 4, insbesondere 2 oder 3 Kohlenstoffatomen, wie Carbomethoxy und Carboäthoxy; Sulfo ($-SO_3H$); Alkylsulfonyl mit vorzugsweise 1 bis 4, insbesondere 1 oder 2 Kohlenstoffatomen, wie Methylsulfonyl und Äthylsulfonyl; Arylsulfonyl mit vorzugsweise 6 oder 10 Arylkohlenstoffatomen, wie Phenylsulfonyl.

Als Alkyl $R^2$ steht geradkettiges oder verzweigtes Alkyl

mit vorzugsweise 1 bis 6, insbesondere 1 bis 4 Kohlenstoffatomen. Beispielshaft seien Methyl, Äthyl, n- und i-Propyl, n-, i- und t-Butyl, genannt.

Als Cycloalkyl $R^2$ steht mono-, bi- und tricyclisches Cycloalkyl mit vorzugsweise 3 bis 10, insbesondere 3, 5 oder 6 Kohlenstoffatomen. Beispielshaft seien Cyclopropyl, Cyclobutyl, Cyclopentyl, Cyclohexyl, Cycloheptyl, Bicyclo /‾2.2.1._7heptyl, Bicyclo /‾2.2.2_7octyl und Adamantyl genannt.

Als Cycloalkylalkyl $R^2$ steht Cycloalkylalkyl mit vorzugsweise 3 bis 10, insbesondere 3, 5 oder 6 Kohlenstoffatomen im Cycloalkylteil und vorzugsweise 1 bis 6, insbesondere 1 bis 4 Kohlenstoffatomen im Alkylteil. Beispielshaft seien Cyclopropylmethyl, Cyclopentylmethyl, Cyclohexylmethyl, Cyclopropyläthyl, Cyclopentyläthyl und Cyclohexyläthyi genannt.

Als Aralkyl $R^2$ steht Aralkyl mit vorzugsweise 6 oder 10, insbesondere 6 Kohlenstoffatomen im Arylteil und vorzugsweise 1 bis 4, insbesondere 1 oder 2 Kohlenstoffatomen im Alkylteil, wobei der Alkylteil geradkettig oder verzweigt sein kann. Beispielshaft seien Benzyl und Phenyläthyl genannt.

Als $R^3$-Alkyl-($C_1$-$C_2$) in der Formel III steht Methyl, Äthyl, n-Propyl, i-Propyl, n-Butyl, i-Butyl, t-Butyl, insbesondere Methyl und Äthyl.

Als Alkyl $R^4$ steht geradkettiges oder verzweigtes Alkyl mit vorzugsweise 1 bis 6, insbesondere 1 bis 4 Kohlenstoffatomen. Beispielshaft seien Methyl, Äthyl, n- und i-Propyl, n-, i- und t-Butyl genannt.

Als Alkoxy $R^4$ steht geradkettiges oder verzweigtes Alkoxy mit vorzugsweise 1 bis 6, insbesondere 1 bis 4 Kohlenstoffatomen. Beispielshaft seien Methyl, Äthoxy, n- und i-Propoxy und n-, i- und t-Butoxy genannt.

Unter den Monocarboxylaten von Phenylguanidinsulfonsäureestern der Formel I haben sich als Anthelminthica diejenigen in besonderem Maße bewährt, in welchen die Substituenten R Wasserstoff, Chlor, Trifluormethyl oder -CN, $R^1$ Wasser-

stoff, Methyl, Äthyl, Propyl, iso-Propyl oder Methoxymethyl, und $R^2$ Wasserstoff bedeuten.

Die als Ausgangsmaterialien verwendeten substituierten Anilinderivate der Formel II und die Phenylguanidinderivate der Formel IV sowie Verfahren zu ihrer Herstellung sind aus der DT-OS 26 08 238 bekannt.
Die Isothioharnstoffe der Formel III sind zum Teil bekannt. Sie können durch Umsetzung von gegebenenfalls substituierten S-Alkylisothioharnstoffen mit Chlorameisensäuremethylester erhalten werden.
Im einzelnen seien beispielshaft folgende Isothioharnstoffe der Formel III genannt:
N-Methoxycarbonyl-S-methyl-isothioharnstoff
N-Methoxycarbonyl-N'-methyl-S-methyl-isothioharnstoff
N-Methoxycarbonyl-N'-äthyl-S-methyl-isothioharnstoff
N-Methoxycarbonyl-N'-propyl-S-methyl-isothioharnstoff
N-Methoxycarbonyl-N'-isopropyl-S-methyl-isothioharnstoff
N-Methoxycarbonyl-N'-butyl-S-methyl-isothioharnstoff
N-Methoxycarbonyl-N'-isobutyl-S-methyl-isothioharnstoff
N-Methoxycarbonyl-N'-tert. butyl-S-methyl-isothioharnstoff
N-Methoxycarbonyl-N'-cyclohexyl-S-methyl-isothioharnstoff
N-Methoxycarbonyl-N'-cyclohexylmethyl-S-methyl-isothioharnstoff
N-Methoxycarbonyl-N'-benzyl-S-methyl-isothioharnstoff
N-Methoxycarbonyl-N'-α-phenäthyl-S-methyl-isothioharnstoff
N-Methoxycarbonyl-N'-ß-phenäthyl-S-methyl-isothioharnstoff

Die O-Methylisoharnstoffe der Formel IIIa können durch Umsetzung von entsprechenden O-Alkylisoharnstoffen mit Chlorameisensäuremethylester erhalten werden.

Als Lösungsmittel kommen bei der Umsetzung von Verbindungen der Formel II mit Stoffen der Formel III bzw. IIIa alle polaren organischen Lösungsmittel in Frage, vorzugsweise Alkohole, wie Methanol, Isopropanol, sowie deren Gemische mit Wasser, Ketone, wie Aceton, sowie deren Gemische mit Wasser, aber auch Äther wie Dioxan oder Tetrahydrofuran.

Bei der Herstellung der Verbindungen der Formel I aus Verbindungen der Formel II mit Stoffen der Formel III bzw. IIIa können als Katalysatoren beliebige organische oder anorganische Säuren verwendet werden. Vorteilhaft verwendet man leicht zugängliche Säuren wie Salzsäure, Schwefelsäure, Salpetersäure, Ameisensäure, Essigsäure, p-Toluolsulfonsäure.

Die Reaktionstemperaturen bei der Herstellung können in einem Bereich variiert werden. Im allgemeinen arbeitet man zwischen 0°C und 120°C, vorzugsweise zwischen 10° und 30°C. Die Umsetzung wird im allgemeinen bei Normaldruck durchgeführt.

Die Umsetzung von Verbindungen der Formel II mit solchen der Formel III bzw. IIIa erfolgt zweckmäßig in äquimolaren Mengen.

Die Hydrolyse der Verbindungen der Formel IV wird zweckmäßig durch Erhitzen in einem Alkohol, wie Methanol, Äthanol, Isopropanol sowie deren Gemischen mit Wasser in Gegenwart einer Base, z. B. eines aliphatischen Amins, wie Methylamin, Butylamin, Piperidin oder N-Methyl-piperazin, eines Alkoholats wie Natriummethylat oder in Gegenwart einer anorganischen Base wie KOH oder NaOH durchgeführt. Als besonders geeignet haben sich Lösungen von Butylamin in Methanol oder Äthanol erwiesen. Die Hydrolyse gelingt auch durch längeres Erhitzen mit Wasser in Gegenwart eines Lösungsmittels wie Aceton.

Die Reaktionstemperaturen der Hydrolysereaktion können in einem größeren Bereich variiert werden, im allgemeinen arbeitet man zwischen 0°C und 120°C, vorzugsweise zwischen 40°C und 100°C. Die Umsetzung wird im allgemeinen bei Normaldruck durchgeführt. Sie ist im allgemeinen im basischen Bereich schon nach wenigen Minuten beendet.

Die Monocarboxylate von Phenylguanidinsulfonsäureestern der Formel I umfassen beispielsweise folgende Verbindungen:

N-(2-Acetamido-5-phenylsulfonyloxy-phenyl)-N'-methoxycarbonyl-guanidin

N-(2-Formamido-5-phenylsulfonyloxy-phenyl)-N'-methoxycarbonyl-guanidin

N-(2-Propionamido-5-phenylsulfonyloxy-phenyl)-N'-methoxycarbonyl-guanidin

N-(2-Butyramido-5-phenylsulfonyloxy-phenyl)-N'-methoxycarbonyl-guanidin

N-(2-iso-Butyramido-5-phenylsulfonyloxy-phenyl)-N'-methoxycarbonyl-guanidin

N-(2-Valeramido-5-phenylsulfonyloxy-phenyl)-N'-methoxycarbonyl-guanidin

N-(2-iso-Valeramido-5-phenylsulfonyloxy-phenyl)-N'-methoxycarbonyl-guanidin

N-(2-Capronamido-5-phenylsulfonyloxy-phenyl)-N'-methoxycarbonyl-guanidin

N-(2-iso-Capronamido-5-phenylsulfonyloxy-phenyl)-N'-methoxycarbonyl-guanidin

N-(2-Methoxyacetamido-5-phenylsulfonyloxy-phenyl)-N'-methoxycarbonyl-guanidin

N-(2-Acetamido-5-(4-chlor-phenylsulfonyloxy)-phenyl)-N'-methoxycarbonyl-guanidin

N-(2-Acetamido-5-(3-chlor-phenylsulfonyloxy)-phenyl)-N'-methoxycarbonyl-guanidin

N-(2-Acetamido-5-(2-chlor-phenylsulfonyloxy)-phenyl)-N'-methoxycarbonyl-guanidin

N-(2-Methoxyacetamido-5-(3-chlor-phenylsulfonyloxy)-phenyl)-N'-methoxycarbonyl-guanidin

N-(2-Methoxyacetamido-5-(3-brom-phenylsulfonyloxy)-phenyl)-N'-methoxycarbonyl-guanidin

N-(2-Methoxyacetamido-5-(3-methyl-phenylsulfonyloxy)-phenyl)-N'-methoxycarbonyl-guanidin

N-(2-Methoxyacetamido-5-(3-tert.-butyl-phenylsulfonyloxy)-phenyl)-N'-methoxycarbonyl-guanidin

N-(2-Methoxyacetamido-5-(3-methoxy-phenylsulfonyloxy)-phenyl)-N'-methoxycarbonyl-guanidin

N-(2-Methoxyacetamido-5-(3-äthoxy-phenylsulfonyloxy)-phenyl)-N'-methoxycarbonyl-guanidin

N-(2-Methoxyacetamido-5-(3-propoxy-phenylsulfonyloxy)-phenyl)-N'-methoxycarbonyl-guanidin

N-(2-Methoxyacetamido-5-(3-isopropoxy-phenylsulfonyloxy)-phenyl)-N'-methoxycarbonyl-guanidin

N-(2-Methoxyacetamido-5-(3-butoxy-phenylsulfonyloxy)-phenyl)-N'-methoxycarbonyl-guanidin

N-(2-Methoxyacetamido-5-(3-isobutoxy-phenylsulfonyloxy)-phenyl)-N'-methoxycarbonyl-guanidin

N-(2-Methoxyacetamido-5-(4-cyano-phenylsulfonyloxy)-phenyl)-N'-methoxycarbonyl-guanidin

N-(2-Methoxyacetamido-5-(3-trifluormethyl-phenylsulfonyloxy)-phenyl)-N'-methoxycarbonyl-guanidin

N-(2-Methoxyacetamido-5-(3-chlor-4-methyl-phenylsulfonyloxy)-phenyl)-N'-methoxycarbonyl-guanidin

N-(2-Propionamido-5-(3-trifluormethyl-phenylsulfonyloxy)-phenyl-N'-methoxycarbonyl-guanidin

N-(2-Butyramido-5-(3-trifluormethyl-phenylsulfonyloxy)-phenyl-N'-methoxycarbonyl-guanidin

N-(2-Formamido-5-(3-trifluormethyl-phenylsulfonyloxy)-phenyl-N'-methoxycarbonyl-guanidin

N-(2-Methoxyacetamido-5-phenylsulfonyloxy-phenyl)-N'-methoxycarbonyl-N"-methyl-guanidin

N-(2-Methoxyacetamido-5-phenylsulfonyloxy-phenyl)-N'-methoxycarbonyl-N"-butyl-guanidin

N-(2-Methoxyacetamido-5-phenylsulfonyloxy-phenyl)-N'-methoxycarbonyl-N"-cyclohexylguanidin

N-(2-Formamido-5-phenylsulfonyloxy-phenyl)-N'-methoxycarbonyl-N"-methylguanidin

N-(2-Propionamido-5-phenylsulfonyloxy-phenyl)-N'-methoxycarbonyl-N"-methylguanidin

N-(2-Butyramido-5-phenylsulfonyloxy-phenyl)-N'-methoxycarbonyl-N"-methylguanidin

N-(2-Acetamido-5-phenoxysulfonyl-phenyl)-N'-meth

N-(2-Butyramido-5-phenoxysulfonyl-phenyl)-N'-methoxycarbonyl-guanidin

N-(2-iso-Butyramido-5-phenoxysulfonyl-phenyl)-N'-methoxycarbonyl-guanidin

N-(2-Valeramido-5-phenoxysulfonyl-phenyl)-N'-methoxycarbonyl-guanidin

N-(2-iso-Valeramido-5-phenoxysulfonyl-phenyl)-N'-methoxycarbonyl-guanidin

N-(2-Capronamido-5-phenoxysulfonyl-phenyl)-N'-methoxycarbonyl-guanidin

N-(2-iso-Capronamido-5-phenoxysulfonyl-phenyl)-N'-methoxycarbonyl-guanidin

N-(2-Methoxyacetamido-5-phenoxysulfonyl-phenyl)-N'-methoxycarbonyl-guanidin

N-(2-Acetamido-5-(4-chlor-phenoxysulfonyl)-phenyl)-N'-methoxycarbonyl-guanidin

N-(2-Acetamido-5-(3-chlor-phenoxysulfonyl)-phenyl)-N'-methoxycarbonyl-guanidin

N-(2-Acetamido-5-(2-chlor-phenoxysulfonyl)-phenyl)-N'-methoxycarbonyl-guanidin

N-(2-Methoxyacetamido-5-(3-chlor-phenoxysulfonyl)-phenyl)-N'-methoxycarbonyl-guanidin

N-(2-Methoxyacetamido-5-(3-brom-phenoxysulfonyl)-phenyl)-N'-methoxycarbonyl-guanidin

N-(2-Methoxyacetamido-5-(3-methyl-phenoxysulfonyl)-phenyl)-N'-methoxycarbonyl-guanidin

N-(2-Methoxyacetamido-5-(3-tert.-butyl-phenoxysulfonyl)-phenyl)-N'-methoxycarbonyl-guanidin

N-(2-Methoxyacetamido-5-(3-methoxy-phenoxysulfonyl)-phenyl)-N'-methoxycarbonyl-guanidin

N-(2-Methoxyacetamido-5-(3-äthoxy-phenoxysulfonyl)-phenyl)-N'-methoxycarbonyl-guanidin

N-(2-Methoxyacetamido-5-(3-propoxy-phenoxysulfonyl)-phenyl)-N'-methoxycarbonyl-guanidin

N-(2-Methoxyacetamido-5-(3-isopropoxy-phenoxysulfonyl)-phenyl)-N'-methoxycarbonyl-guanidin

N-(2-Methoxyacetamido-5-(3-butoxy-phenoxysulfonyl)-phenyl)-N'-methoxycarbonyl-guanidin

N-(2-Methoxyacetamido-5-(3-isobutoxy-phenoxysulfonyl)-phenyl)-N'-methoxycarbonyl-guanidin

N-(2-Methoxyacetamido-5-(4-cyano-phenoxysulfonyl)-phenyl)-N'-methoxycarbonyl-guanidin

N-(2-Methoxyacetamido-5-(3-trifluormethyl-phenoxysulfonyl)-phenyl)-N'-methoxycarbonyl-guanidin

N-(2-Methoxyacetamido-5-(3-chlor-4-methyl-phenoxysulfonyl)-phenyl)-N'-methoxycarbonyl-guanidin

N-(2-Propionamido-5-(3-trifluormethyl-phenoxysulfonyl)-phenyl)-N'-methoxycarbonyl-guanidin

N-(2-Butyramido-5-(3-trifluormethyl-phenoxysulfonyl)-phenyl)-N'-methoxycarbonyl-guanidin

N-(2-Formamido-5-(3-trifluormethyl-phenoxysulfonyl)-phenyl)-N'-methoxycarbonyl-guanidin

N-(2-Methoxyacetamido-5-phenoxysulfonyl-phenyl)-N'-methoxycarbonyl-N"-methyl-guanidin

N-(2-Methoxyacetamido-5-phenoxysulfonyl-phenyl)-N'-methoxycarbonyl-N"-butyl-guanidin

N-(2-Methoxyacetamido-5-phenoxysulfonyl-phenyl)-N'-methoxycarbonyl-N"-cyclohexyl-guanidin

N-(2-Formamido-5-phenoxysulfonyl-phenyl)-N'-methoxycarbonyl-N"-methyl-guanidin

N-(2-Propionamido-5-phenoxysulfonyl-phenyl)-N'-methoxycarbonyl-N"-methyl-guanidin

N-(2-Butyramido-5-phenoxysulfonyl-phenyl)-N'-methoxycarbonyl-N"-methyl-guanidin

Die Monocarboxylate der Phenylguanidinsulfonsäureester der Formel I gemäß der Erfindung sind wertvolle Chemotherapeutica und eignen sich zur Bekämpfung von parasitären Erkrankungen bei Mensch und Tier, insbesondere von Helminthen und Leberegeln.
Sie sind besonders wirksam gegen eine große Anzahl von Helminthen, z. B. Haemonchus, Trichostrongylus, Ostertagia, Strongyloides, Cooperia, Chabertia, Oesophagostomum, Hyostrongulus, Ankylostoma, Askaris und Heterakis und Fasciola. Besonders ausgeprägt ist die Wirksamkeit gegenüber Magen-Darmstrongyliden und Leberegeln, von denen vor allem

Wiederkäuer befallen werden. Deshalb werden die Verbindungen gemäß der Erfindung insbesondere in Tierarzneimitteln verwendet.

Die erfindungsgemäßen Verbindungen der Formel I können im Gegensatz zu den nach DT-OS 21 17 293, 22 50 911, 23 04 764 und 24 23 679 bekannten Verbindungen vorteilhaft parenteral appliziert werden und wirken im Gegensatz zu den nach DT-OS 26 30 847 bekannten Verbindungen überraschenderweise sowohl gegen Nemathoden als auch gegen Leberegel.

Die Verbindungen der Formel I werden je nach Lage des Falles in Dosierungen zwischen 0,5 und 50 mg pro kg Körpergewicht 1 bis 14 Tage lang verabreicht.

Zur oralen Applikation kommen Tabletten, Dragees, Kapseln, Pulver, Granulate oder Pasten in Betracht, welche die Wirkstoffe zusammen mit üblichen Hilfs- oder Trägerstoffen wie Stärke, Cellulosepulver, Talcum, Magnesiumstearat,Zukker, Gelatine, Calciumcarbonat, feinverteilter Kieselsäure, Carboxymethylcellulose oder ähnlichen Stoffen enthalten.

Die Verfahrensprodukte sind nicht nur oral appliziert ausgezeichnet wirksam, sondern wirken auch parenteral.

Zur parenteralen Applikation löst man entweder eine Verbindung der Formel I in einem Äquivalent einer anorganischen oder organischen Säure, oder man löst ein Salz dieser Verbindungen in Wasser.

Die Möglichkeit der parenteralen Applikation der Verbindungen der Formel I stellt einen erheblichen Fortschritt in der anthelmintischen Behandlung von Tieren, wie Rindern, Pferden, Schweinen, Schafen, Hunden, Katzen usw. dar. Sie ist besonders geeignet für die Massenbehandlung von Großtieren, wie Rindern und Pferden, insbesondere,wenn diese Tiere gleichzeitig mit Leberegeln infiziert sind.

Herstellungsbeispiele

Verfahren $a_1$)

Beispiel 1.1

Man erhitzt ein Gemisch von 3,4 g 2-Methoxyacetamido-5-phenylsulfonyloxy-anilin (Formel II), 25 ml Methanol, 3 ml Eisessig und 2 g N-Methoxycarbonyl-S-methyl-isothioharnstoff (Formel III) einen Tag lang am Rückfluß. Nach Stehen über Nacht bei Zimmertemperatur filtriert man das ausgeschiedene N-(2-Methoxyacetamido-5-phenylsulfonyloxy-phenyl)-N'-methoxycarbonyl-guanidin (Formel I) ab. Ausbeute 0,4 g; F.P. 190°C.

In analoger Arbeitsweise erhält man aus substituierten 2-Acylamido-anilinderivaten (Formel II) und N-substituierten N-Methoxycarbonyl-S-methyl-isothioharnstoffen (Formel III):

1.2 N-(2-Formamido-5-phenylsulfonyloxy-phenyl)-N'-methyl-N"-methoxycarbonyl-guanidin

1.3 N-(2-Acetamido-5-phenylsulfonyloxy-phenyl)-N'-methyl-N"-methoxycarbonyl-guanidin

1.4 N-(2-Methoxyacetamido-5-phenylsulfonyloxy-phenyl)-N'-methyl-N"-methoxycarbonyl-guanidin

1.5 N-(2-Propionamido-5-phenylsulfonyloxy-phenyl)-N'-methyl-N"-methoxycarbonyl-guanidin

1.6 N-(2-Methoxyacetamido-5-phenylsulfonyloxy-phenyl)-N'-äthyl-N"-methoxycarbonyl-guanidin

1.7 N-(2-Methoxyacetamido-5-phenylsulfonyloxy-phenyl)-N'-propyl-N"-methoxycarbonyl-guanidin

1.8 N-(2-Methoxyacetamido-5-phenylsulfonyloxy-phenyl)-N'-butyl-N"-methoxycarbonyl-guanidin

1.9 N-(2-Methoxyacetamido-5-phenylsulfonyloxy-phenyl)-N'-isobutyl-N"-methoxycarbonyl-guanidin

1.10 N-(2-Methoxyacetamido-5-phenylsulfonyloxy-phenyl)-N'-cyclohexyl-N"-methoxycarbonyl-guanidin

1.11 N-(2-Methoxyacetamido-5-phenylsulfonyloxy-phenyl)-N'-cyclohexylmethyl-N"-methoxycarbonyl-guanidin

1.12 N-(2-Methoxyacetamido-5-phenylsulfonyloxy-phenyl)-N'-benzyl-N"-methoxycarbonyl-guanidin

Verfahren $a_2$)

Beispiel 2

Man erhitzt ein Gemisch von 3,4 g 2-Methoxyacetamido-5-phenylsulfonyloxy-anilin (Formel II), 25 ml Methanol, 3 ml Eisessig und 2 g N-Methoxycarbonyl-O-methyl-isoharnstoff (Formel IIIa) einen Tag lang am Rückfluß. Nach Stehen über Nacht bei Zimmertemperatur filtriert man die Flüssigkeit von ausgeschiedenen Verunreinigungen ab, dampft das Filtrat zur Trockne ein, nimmt den Rückstand in Essigester auf, wäscht mehrmals mit Wasser und engt das Lösungsmittel nach dem Trocknen mit $MgSO_4$ unter vermindertem Druck ein. Durch Verrühren des Rückstandes mit 0,5 n HCl bei Zimmertemperatur und Fällung des Filtrates mit Ammoniak wird das gebildete N-(2-Methoxyacetamido-5-phenyloxy-phenyl)-N'-methoxycarbonyl-guanidin (Formel I) in reiner Form erhalten. Es ist nach Dünnschichtchromatographie identisch mit dem nach Beispiel 1 erhaltenen Verfahrensprodukt.

Herstellung des Ausgangsmaterials der Formel IIIa:

Man löst 246 g O-Methyl-isoharnstoffsulfat in 300 ml Wasser und tropft bei 10°C 163 ml Chlorameisensäuremethylester zu und anschließend 170 g NaOH, gelöst in 510 ml Wasser. Man rührt weitere 3 Stunden bei Zimmertemperatur. Dann extrahiert man die Reaktionslösung mit Essigester und trocknet diesen Extrakt mit wasserfreiem Natriumsulfat. Nach Eindampfen unter vermindertem Druck bei einer maximalen Badtemperatur von 40°C erhält man den N-Methoxycarbonyl-O-methyl-isoharnstoff als Öl, das allmählich zu Kristallen mit einem F.P. von 40°C erstarrt. Ausbeute 150.

Verfahren b) (Hydrolyse)

Beispiel 3

Man rührt ein Gemisch von 2 g N-(2-Methoxyacetamido-5-phenylsulfonyloxy-phenyl)-N',N"-bis-methoxycarbonylguanidin (Formel IV), 20 ml Methanol und 2 ml Butylamin 10 Minuten bei 50°C. Schon nach zwei Minuten ist eine klare Lösung entstanden, aus der kurz darauf das N-(2-Methoxyacetamido

5-phenylsulfonyloxy-phenyl)-N'-methoxycarbonyl-guanidin (Formel I) ausfällt. Man läßt die Reaktionsmischung einige Stunden bei 10°C stehen, filtriert die Fällung ab und wäscht sie mit Methanol. Ausbeute 1.7 g; F.P. 190°C (Zersetzung). Das Verfahrensprodukt ist mit dem nach Beispiel 1 erhaltenen identisch.

Das für die Durchführung der Reaktion erforderliche N-(2-methoxyacetamido-5-phenylsulfonyloxy-phenyl)-N'-,N"-bis-methoxycarbonyl-guanidin (Formel IV) wird nach der DT-OS 26 08 238, Beispiel 5.2 erhalten.

Anstelle von Butylamin kann man in gleicher Arbeitstechnik auch eine 40%ige wässrige Methylaminlösung, Piperidin oder N-Methyl-piperazin einsetzen. Entsprechende Resultate werden auch mit Natronlauge, Ammoniak oder Natriummethylat in alkoholischer Lösung erhalten.

<u>Beispiel 4 (noch Verfahren b) )</u>

Analog Beispiel 3 werden die in der nachstehenden Tabelle zusammengefaßten Verbindungen der Formel I aus den entsprechenden Ausgangsmaterialien der Formel IV dargestellt, in welchen X jeweils $-SO_2-O-$ und $R^4$ $-OCH_3$ bedeutet:

| Ausgangsmaterial der Formel IV | | | | Schmelzpunkt [°C] des Verfahrensproduktes der Formel I |
|---|---|---|---|---|
| | $R^1$ | R | bekannt aus Beispiel Nr. der DT-OS 26 08 238 | |
| 2.1 | $CH_3$ | H | 4.1 | 175 |
| 2.2 | $C_2H_5$ | H | 5.3 | 149 |
| 2.3 | $C_3H_7$ | H | 5.1 | 148 |
| 2.4 | $iC_3H_7$ | H | 5.4 | |
| 2.5 | $C_4H_9$ | H | 5.5 | |
| 2.6 | $iC_4H_9$ | H | 5.6 | |

| Ausgangsmaterial der Formel IV | | | | Schmelzpunkt /‾°C_7 des Verfahrensproduktes der Formel I |
|---|---|---|---|---|
| | $R^1$ | R | bekannt aus Beispiel Nr. der DT-OS 26 08 238 | |
| 2.7 | $C_5H_{11}$ | H | 5.7 | |
| 2.8 | $iC_5H_{11}$ | H | 5.8 | |
| 2.9 | cyclo$C_5H_9$ | H | 5.9 | |
| 2.10 | cyclo$C_6H_{11}$ | H | 5.10 | |
| 2.11 | $CH_2C_6H_5$ | H | 5.11 | |
| 2.12 | $CH_2OC_6H_5$ | H | 5.12 | |
| 2.13 | $CH_3$ | 4-Cl | 6.1 | |
| 2.14 | $CH_3$ | 3-Cl | 6.2 | |
| 2.15 | $C_3H_7$ | 3-Cl | 6.10 | |
| 2.16 | $CH_2OCH_3$ | 3-Cl | 6.11 | |
| 2.17 | $C_3H_7$ | 2-Cl | 6.12 | |
| 2.18 | $CH_3$ | 2,5-Cl | 6.13 | |
| 2.19 | $C_3H_7$ | 3,5-Cl | 6.14 | |
| 2.10 | $CH_3$ | 4-Br | 6.15 | |
| 2.21 | $C_2H_5$ | 3-Br | 6.16 | |
| 2.22 | $C_3H_7$ | 3-Br | 6.17 | |
| 2.23 | $CH_2OCH_3$ | 3-Br | 6.18 | |
| 2.24 | $CH_3$ | 2-Br | 6.19 | |
| 2.25 | $CH_3$ | 4-$CH_3$ | 6.20 | |
| 2.26 | $CH_3$ | 3-$CH_3$ | 6.21 | |
| 2.27 | $C_2H_5$ | 3-$CH_3$ | 6.22 | |
| 2.28 | $C_3H_7$ | 3-$CH_3$ | 6.23 | |
| 2.29 | $CH_2OCH_3$ | 3-$CH_3$ | 6.24 | |
| 2.30 | $CH_3$ | 2-$CH_3$ | 6.25 | |
| 2.31 | $CH_3$ | 4-t.Butyl | 6.26 | |
| 2.32 | $CH_3$ | 2,4-$CH_3$ | 6.27 | |
| 2.33 | $CH_3$ | 2-Cl-4-$CH_3$ | 6.28 | |
| 2.34 | $CH_3$ | 3,4-Cl | 6.29 | |

| Ausgangsmaterial der Formel IV | | | | Schmelzpunkt /°C/ des Verfahrensproduktes der Formel I |
|---|---|---|---|---|
| | $R^1$ | R | bekannt aus Beispiel Nr. der DT-OS 26 08 238 | |
| 2.35 | $CH_3$ | 3-$CF_3$ | 6.30 | 182 |
| 2.36 | $CH_2OCH_3$ | 3-$CF_3$ | 6.31 | 189 |
| 2.37 | $C_2H_5$ | 3-$CF_3$ | 6.32 | 154 |
| 2.38 | $C_3H_7$ | 3-$CF_3$ | 6.33 | 168 |
| 2.39 | $CH_3$ | 4-$OCH_3$ | 6.38 | |
| 2.40 | $CH_3$ | 3-$OCH_3$ | 6.39 | |
| 2.41 | $CH_3$ | 3-$OC_2H_5$ | 6.40 | |
| 2.42 | $CH_3$ | 3-CN | 6.41 | |
| 2.43 | $C_2H_5$ | 3-CN | 6.42 | |
| 2.44 | $C_3H_7$ | 3-CN | 6.43 | |
| 2.45 | $CH_2OCH_3$ | 3-CN | 6.44 | |
| 2.46 | $C_6H_{11}$ | 3-$CF_3$ | 6.45 | |
| 2.47 | $CH_2OCH_3$ | 3-$OCH_3$ | 6.46 | |
| 2.48 | $C_2H_5$ | 3-$OCH_3$ | 6.47 | |
| 2.49 | H | H | | |

Beispiel 5 (noch Verfahren b) )

Analog Beispiel 3 werden die in der nachstehenden Tabelle zusammengefaßten Verbindungen der Formel I aus den entsprechenden Ausgangsmaterialien der Formel IV dargestellt, in welchen X jeweils $-O-SO_2-$ und $R^4$ $-OCH_3$ bedeutet:

| Ausgangsmaterial der Formel IV | | | | Schmelzpunkt [°C] des Verfahrensproduktes der Formel I |
|---|---|---|---|---|
| | $R^1$ | $R^2$ | bekannt aus Beispiel Nr. der DT-OS 26 08 238 | |
| 3.1 | $CH_3$ | H | 1.1 | 196 |
| 3.2 | $CH_2OCH_3$ | H | 2.2 | 203 |
| 3.3 | $C_2H_5$ | H | 2.3 | 168 |
| 3.4 | $C_3H_7$ | H | 2.1 | 194 |
| 3.5 | $iC_3H_7$ | H | 2.4 | |
| 3.6 | $C_4H_9$ | H | 2.5 | |
| 3.7 | $iC_4H_9$ | H | 2.6 | |
| 3.8 | $C_5H_{11}$ | H | 2.7 | |
| 3.9 | $iC_5H_{11}$ | H | 2.8 | |
| 3.10 | $cycloC_5H_9$ | H | 2.9 | |
| 3.11 | $cycloC_6H_{11}$ | H | 2.10 | |
| 3.12 | $CH_2C_6H_5$ | H | 2.11 | |
| 3.13 | $CH_2OC_6H_5$ | H | 2.12 | |
| 3.14 | $CH_3$ | 4-Cl | 3.1 | |
| 3.15 | $CH_3$ | 3-Cl | 3.2 | |
| 3.16 | $C_3H_7$ | 3-Cl | 3.10 | |
| 3.17 | $CH_2OCH_3$ | 3-Cl | 3.11 | |
| 3.18 | $C_3H_7$ | 2-Cl | 3.12 | |
| 3.19 | $CH_3$ | 2,5-Cl | 3.13 | |
| 3.20 | $C_3H_7$ | 3,5-Cl | 3.14 | |
| 3.21 | $CH_3$ | 4-Br | 3.15 | |
| 3.22 | $C_2H_5$ | 3-Br | 3.16 | |

| Ausgangsmaterial der Formel IV | | | | Schmelzpunkt /¯°C 7 des Verfahrensproduktes der Formel I |
|---|---|---|---|---|
| | $R^1$ | R | bekannt aus Beispiel Nr. der DT-OS 26 08 238 | |
| 3.23 | $C_3H_7$ | 3-Br | 3.17 | |
| 3.24 | $CH_2OCH_3$ | 3-Br | 3.18 | |
| 3.25 | $CH_3$ | 2-Br | 3.19 | |
| 3.26 | $CH_3$ | 4-$CH_3$ | 3.20 | |
| 3.27 | $CH_3$ | 3-$CH_3$ | 3.21 | |
| 3.28 | $C_2H_5$ | 3-$CH_3$ | 3.22 | |
| 3.29 | $C_3H_7$ | 3-$CH_3$ | 3.23 | |
| 3.30 | $CH_2OCH_3$ | 3-$CH_3$ | 3.24 | |
| 3.31 | $CH_3$ | 2-$CH_3$ | 3.25 | |
| 3.32 | $CH_3$ | 4-t.Butyl | 3.26 | |
| 3.33 | $CH_3$ | 2,4-$CH_3$ | 3.27 | |
| 3.34 | $CH_3$ | 2-Cl-4-$CH_3$ | 3.28 | |
| 3.35 | $CH_3$ | 3,5-Cl | 3.29 | |
| 3.36 | $CH_3$ | 3-$CF_3$ | | 186 |
| 3.37 | $CH_2OCH_3$ | 3-$CF_3$ | 3.30 | |
| 3.38 | $C_2H_5$ | 3-$CH_3$ | 3.31 | |
| 3.39 | $C_3H_7$ | 3-$CF_3$ | 3.32 | |
| 3.40 | $CH_3$ | 4-$OCH_3$ | 3.37 | |
| 3.41 | $CH_3$ | 3-$OCH_3$ | 3.38 | |
| 3.42 | $CH_3$ | 3-$OC_2H_5$ | 3.39 | |
| 3.43 | $CH_3$ | 3-CN | 3.40 | |
| 3.44 | $C_2H_5$ | 3-CN | 3.41 | |
| 3.45 | $C_3H_7$ | 3-CN | 3.42 | |
| 3.46 | $CH_2OCH_3$ | 3-CN | 3.43 | |

Das für die Darstellung des Verfahrensproduktes nach Beispiel 3.36 benötigte N-(2-Acetamido-5-(3-trifluormethyl-phenylsulfonyloxy)-phenyl-N',N"-bis-methoxycarbonylgua-

nidin (Formel IV) vom F.P. 152° wird entsprechend dem Beispiel 3 der DT-OS 26 08 238 aus 2-Nitro-4-(3-trifluormethyl-phenoxysulfonyl)-chlorbenzol vom F.P. 65°C über 2-Nitro-4-(3-trifluormethyl-phenoxysulfonyl)-anilin vom F.P. 130°C, 2-Nitro-4-(3-trifluormethyl-phenoxysulfonyl)-anilin vom F.P. 130°C und 2-Nitro-4-(3-trifluormethyl-phenoxysulfonyl)-acetanilid vom F.P. 114°C sowie 2-Amino-4-(3-trifluormethyl-phenoxysulfonyl)-acetanilid vom F.P. 141°C erhalten.

Beispiel 6 (noch Verfahren b) )

Man erhitzt 2,5 g N-(2-Methoxyacetamido-5-phenylsulfonyloxy-phenyl)-N',N"-bis-methoxycarbonylguanidin (Formel IV) in 25 ml Aceton und 25 ml Wasser 24 Stunden im Autoklaven auf 90°C. Nach dem Erkalten filtert man das Rohprodukt ab. Es wird zur Reinigung in 25 ml 0,5 n HCl bei Zimmertemperatur mehrmals verrührt, vom Ungelösten jeweils abfiltriert und die Filtrate mit Ammoniak versetzt. Nach dem Filtern und Trocknen des Rückstandes erhält man 0,5 g reines N-(2-Methoxyacetamido-5-phenylsulfonyloxy-phenyl)-N'-methoxycarbonyl-guanidin vom F.P. 190°C (Zersetzung), das identisch mit dem nach Beispiel 1 erhältlichen Verfahrensprodukt ist.

Beispiel 7 Salzbildung)

Man verrührt 24 g des nach Beispiel 1 erhältlichen freien N-(2-Methoxyacetamino-5-phenylsulfonyloxy-phenyl)-N'-methoxycarbonylguanidin in 250 ml Methanol und macht mit alkoholischer Chlorwasserstofflösung kongosauer. Darauf wird die Lösung unter vermindertem Druck zur Trockne eingedampft, der Rückstand mit Äther versetzt, und das Hydrochlorid des N-(2-Methoxyacetamido-5-phenylsulfonyloxy-phenyl)-N'-methoxycarbonylguanidins abfiltriert. Nach dem Waschen mit Äther und Trocknen unter vermindertem Druck über Ätznatron beträgt die Ausbeute 25 g.
In analoger Weise werden die Hydrochloride der übrigen Verfahrensprodukte erhalten, wie sie in den Beispielen 2 bis 6 beschrieben werden.

1. Monocarboxylate von Phenylguanidinsulfonsäureestern der Formel I

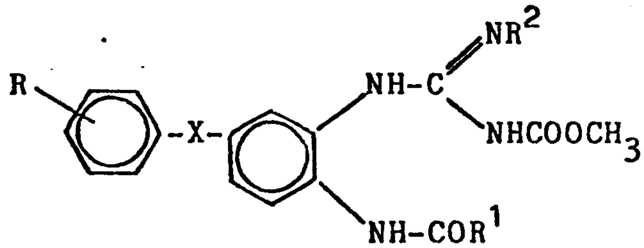

(I)

in welcher

X $-OSO_2-$ oder $-SO_2O-$,

R Wasserstoff, Halogen, Alkyl mit 1 - 4 C-Atomen, Alkoxy mit 1 - 4 C-Atomen, -CN oder $-CF_3$,

$R^1$ Wasserstoff, gegebenenfalls substituiertes Alkyl, gegebenenfalls substituiertes Alkoxy, gegebenenfalls substituiertes Alkoxyalkyl oder gegebenenfalls substituiertes Aralkyl und

$R^2$ Wasserstoff, Alkyl, Cycloalkyl, Cycloalkyl-alkyl oder Aralkyl bedeuten,

und ihre physiologisch verträglichen Salze mit organischen oder anorganischen Säuren.

2. Verfahren zur Herstellung der Monocarboxylate von Phenylguanidinsulfonsäureestern der Formel I, dadurch gekennzeichnet, daß man

a) ein substituiertes Anilinderivat der Formel II

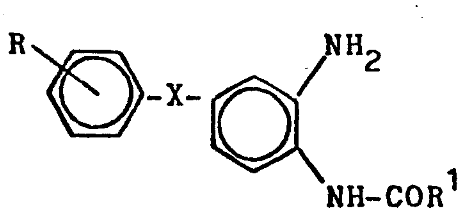

(II)

in welcher X, R und $R^1$ die zu Formel I angegebenen Bedeutungen haben, entweder

$a_1$) mit einem Isothioharnstoffderivat der Formel III

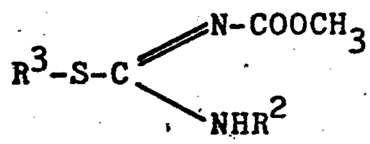

(III)

in welcher $R^2$ die zu Formel I angegebene Bedeutung hat und $R^3$ für Alkyl mit 1 - 4 C-Atomen steht,

oder

$a_2$) mit einem O-Methylisoharnstoffderivat der Formel IIIa

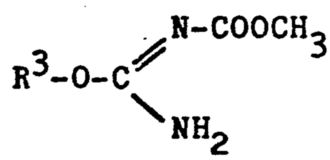

(IIIa)

in welcher $R^3$ für Alkyl mit 1 - 4 C-Atomen steht, in Gegenwart eines Lösungsmittels und gegebenenfalls in Gegenwart einer Säure umsetzt,
oder

b) ein Phenylguanidin-bis-carboxylat der Formel IV

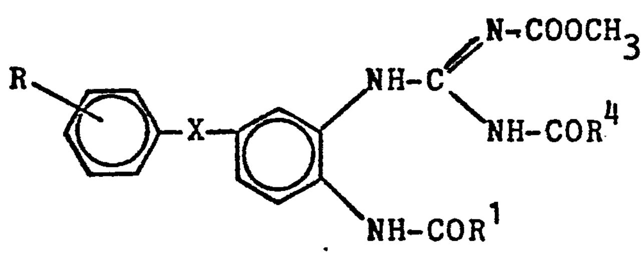

(IV)

in welcher
X, R und $R^1$ die oben angegebenen Bedeutungen haben und $R^4$ für Alkyl oder Alkoxy steht, hydrolysiert,

und gegebenenfalls eine so erhaltene Verbindung der Formel I, in welcher $R^2$ Wasserstoff bedeutet, alkyliert.

3. Arzneimittel, gekennzeichnet durch einen Gehalt an einer Verbindung der Formel I nach Anspruch 1, in Mischung mit einem pharmazeutisch üblichen Trägerstoff und/oder Konstituens.

4. Verwendung einer Verbindung der Formel I nach Anspruch 1 zur Bekämpfung von Helminten in davon befallenen Organismen.

5. Monocarboxylate von Phenylguanidinsulfonsäureestern der Formel I in Anspruch 1, in welcher

X $-SO_2O-$,

R meta-Trifluormethyl,

$R^1$ Methyl, Äthyl, Propyl oder Methoxymethyl und

$R^2$ Wasserstoff bedeuten.

Patentansprüche für Österreich:

1. Verfahren zur Herstellung der Monocarboxylate von Phenylguanidinsulfonsäureestern der Formel I

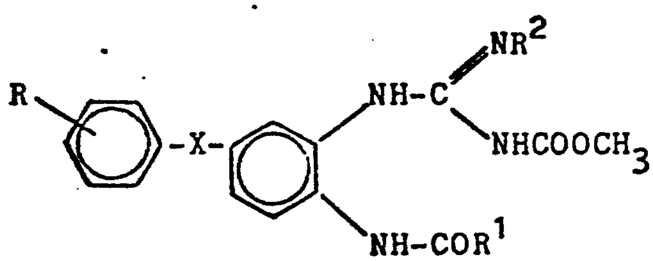

(I)

in welcher

X $-OSO_2-$ oder $-SO_2O-$,

R Wasserstoff, Halogen, Alkyl mit 1 - 4 C-Atomen, Alkoxy mit 1 - 4 C-Atomen, -CN oder $-CF_3$,

$R^1$ Wasserstoff, gegebenenfalls substituiertes Alkyl, gegebenenfalls substituiertes Alkoxy, gegebenenfalls substituiertes Alkoxyalkyl oder gegebenenfalls substituiertes Aralkyl und

$R^2$ Wasserstoff, Alkyl, Cycloalkyl, Cycloalkyl-alkyl oder Aralkyl bedeuten,

und ihre physiologisch verträglichen Salze mit organischen oder anorganischen Säuren, **dadurch gekennzeichnet, daß man**

a) ein substituiertes Anilinderivat der Formel II

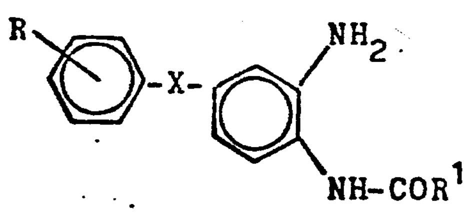

(II)

in welcher X, R und $R^1$ die zu Formel I angegebenen Bedeutungen haben, entweder

$a_1$) mit einem Isothioharnstoffderivat der Formel III

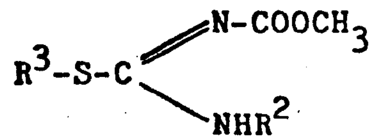

(III)

in welcher $R^2$ die zu Formel I angegebene Bedeutung hat und $R^3$ für Alkyl mit 1 - 4 C-Atomen steht,

oder

$a_2$) mit einem O-Methylisoharnstoffderivat der Formel IIIa

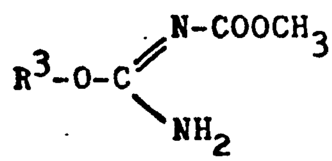

(IIIa)

in welcher $R^3$ für Alkyl mit 1 - 4 C-Atomen steht, in Gegenwart eines Lösungsmittels und gegebenenfalls in Gegenwart einer Säure umsetzt,
oder

b) ein Phenylguanidin-bis-carboxylat der Formel IV

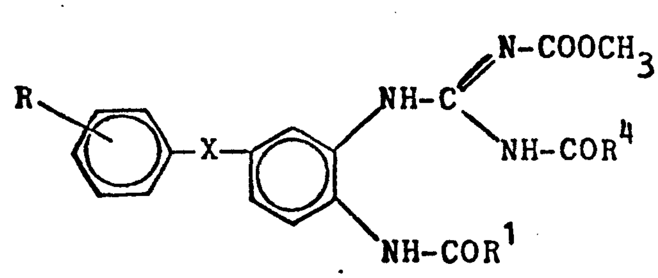

(IV)

in welcher
X, R und $R^1$ die oben angegebenen Bedeutungen haben und $R^4$ für Alkyl oder Alkoxy steht, hydrolysiert,

und gegebenenfalls eine so erhaltene Verbindung der Formel I, in welcher $R^2$ Wasserstoff bedeutet, alkyliert.

2. Verwendung einer Verbindung der Formel I nach Anspruch 1 zur Bekämpfung von Helminten in davon befallenen Organismen.

0008438

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 79 10 2980

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.) |
|---|---|---|---|
| | DE - A - 2 608 238 (HOECHST)<br>* Ansprüche *<br>---- | 1-3,5 | C 07 C 143/68<br>A 61 K 31/325 |

RECHERCHIERTE SACHGEBIETE (Int. Cl.)

C 07 C 143/68

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5
Unvollständig recherchierte Patentansprüche:
Nicht recherchierte Patentansprüche: 4: Verfahren zur chirurgischen oder therapeutischen Behandlung des menschlichen oder tierischen Körpers. (Siehe Art. 52(4) des Europäischen Patentübereinkommens).
Grund für die Beschränkung der Recherche:

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 15-11-1979 | GAUTIER |